# EUROPEAN PATENT SPECIFICATION

(11) **EP 3 062 688 B1**
(45) Date of publication and mention of the grant of the patent: **16.01.2019**
(21) Application number: 14827654.6
(22) Date of filing: 18.12.2014
(51) Int. Cl.: A61B 5/00, A61B 5/042, A61B 5/053

(54) **COAXIAL ELECTRODE CATHETERS FOR EXTRACTING ELECTROPHYSIOLOGIC PARAMETERS**
KOAXIALE ELEKTRODENKATHETER ZUR EXTRAKTION ELEKTROPHYSIOLOGISCHER PARAMETER
CATHÉTERS À ÉLECTRODES COAXIALES POUR EXTRACTION DE PARAMÈTRES ÉLECTROPHYSIOLOGIQUES

(30) Priority: 20.12.2013 US 201361919176 P
(43) Date of publication of application: 07.09.2016
(60) Divisional application: 18205687.9
(73) Proprietor: St. Jude Medical, Cardiology Division, Inc., St. Paul, MN 55117 (US)
(72) Inventor: BYRD, Israel A., Richfield, Minnesota 55423 (US); JUST, Dale E., Minneapolis, Minnesota 55407 (US)
(74) Representative: Kramer Barske Schmidtchen Patentanwälte PartG mbB
(86) International application number: PCT/US2014/071247
(87) International publication number: WO 2015/095577

(56) References cited:
- EP-A1- 1 393 674
- WO-A1-98/49936
- WO-A1-2007/070997
- WO-A2-2008/097767
- US-A1- 2004 010 303
- US-A1- 2012 143 179
- US-A1- 2013 289 369

## Description

### BACKGROUND

The instant disclosure relates to catheters having a plurality of electrodes. In particular, the instant disclosure relates to catheters having a dense collection of small electrodes on the tip of the catheter. The electrodes can be used to derive parameters such as transmembrane current, local conduction velocity, and tissue impedance.

The heart contains two specialized types of cardiac muscle cells. The majority, around ninety-nine percent, of the cardiac muscle cells are contractile cells, which are responsible for the mechanical work of pumping the heart. The second type of cardiac muscle cells are autorhythmic cells, which function as part of the autonomic nervous system to initiate and conduct action potentials responsible for the contraction of the contractile cells. The cardiac muscle displays a pacemaker activity, in which membranes of cardiac muscle cells slowly depolarize between action potentials until a threshold is reached, at which time the membranes fire or produce an action potential. This contrasts with a nerve or skeletal muscle cell, which displays a membrane that remains at a constant resting potential unless stimulated. The action potentials, initiated by the autorhythmic cardiac muscle cells, spread throughout the heart triggering rhythmic beating without any nervous stimulation.

An arrhythmia occurs when the cardiac rhythm becomes irregular, i.e., too fast (tachycardia) or too slow (bradycardia), or the frequency of the atrial and ventricular beats are different. Arrhythmias can develop from either altered impulse formation or altered impulse conduction. Arrhythmias can be either benign or more serious in nature depending on the hemodynamic consequences of arrhythmias and their potential for changing into lethal arrhythmias.

Electrophysiological mapping, and more particularly electrocardiographic mapping, is a part of numerous cardiac diagnostic and therapeutic procedures, such as procedures to treat the foregoing arrhythmias. Typically, such electrophysiology studies employ electrophysiology devices, such as catheters, that include one or more electrodes capable of measuring the electrical activity occurring on the epicardial or endocardial surface, or at other locations on or near the heart. The resultant data set can be used to generate a map of the cardiac electrical activity, which the practitioner can then utilize to develop a course of action (e.g., to identify locations for ablation).

US 2013/0289369 A1 relates to the field of medical devices for achieving intravascular neuro-modulation, wherein a sensor and an electrode are provided as two different components, wherein the electrode is adapted for delivering a thermal electric field for heating of target neural fibers, whereas the sensor is adapted for detecting electrophysiological parameters.

WO 2008/097767 A2 relates to methods and systems for determining information about a position of an object within distribution of materials having different complex conductivities.

EP 1 393 674 A1 relates to an improved catheter that is particularly used for mapping the Purkinje fibers for potential diagnosis of ventricular fibrillation.

### BRIEF SUMMARY

The current disclosure provides solutions to problems with deriving electrophysiological parameters such as transmembrane current, local conduction velocity, and tissue impedance. In general, disclosed herein are catheter systems having a dense collection of small electrodes to measure a large number of surface potentials within a small area. For example, the catheter systems may be used to acquire and analyze electrograms.

One embodiment is an apparatus for use in an electrophysiology procedure such as electrocardiographic mapping, the apparatus including a catheter and a signal processor. The apparatus may be packaged as part of a kit.

The catheter includes a body having a proximal end and a distal tip region. A handle is operably connected to the proximal end of the body. Positioned on the distal tip region are a plurality of electrodes. The electrodes are ring electrodes and dot electrodes (also known as circle or spot electrodes). The electrodes are spot electrodes surrounded by ring electrodes, referred to herein as "coaxtrodes." In other embodiments, the distal tip contains a combination of coaxtrodes and spot electrodes.

The signal processor is operably connected to the plurality of electrodes to receive and analyze electrical signals in order to derive at least one electrophysiological parameter. For example, the electrophysiological parameter may be transmembrane current, tissue impedance, local conduction velocity, and any combinations thereof.

The signal processor has at least one amplifier operably coupled to a plurality of coaxtrodes. In some embodiments, each electrode or coaxtrode is operably coupled to a unique amplifier (*e*.*g*., a one-to-one relationship between electrodes and amplifiers), whereas in other embodiments a plurality of electrodes or coaxtrodes are coupled to each amplifier (*e*.*g*., a many-to-one relationship between electrodes and amplifiers such as multiplexing). In other embodiments, each coaxtrode is connected to the inputs of an instrumentation amplifier. The amplifier(s) may be located at the proximal end of the distal tip region. In other embodiments, the amplifier(s) may be located in the handle.

The apparatus may have a basket-shaped assembly operably connected to the distal tip region. A plurality of spot electrodes and/or coaxtrodes can be located on the basket-shaped assembly.

Another embodiment is an electrophysiology apparatus that includes a catheter and a signal processor. The catheter has a body having a proximal end and a distal end, the distal end having a distal tip. Contained on the distal tip are a plurality of coaxtrodes. A handle is operably connected to the proximal end of the catheter body. The signal processor is operably connected to the plurality of spot electrodes and/or coaxtrodes and derives at least one electrophysiological parameter. The electrophysiological parameter may be, for example, transmembrane current, tissue impedance, and/or local conduction velocity.

The distal tip has a proximal end and a distal end. In some embodiments, at least one amplifier is located at the proximal end of the tip. The at least one amplifier is operably connected to the coaxtrodes. In some embodiments, the amplifier is located in the catheter handle.

The spot electrodes and coaxtrodes are arranged on the distal tip in a pattern that is constant regardless of rotational orientation. The spot electrodes and coaxtrodes form a hexagonal shape and may have an additional spot electrode or coaxtrode positioned in the center of the hexagon.

In some embodiments, the catheter includes a localization element located proximal to the distal tip. The localization element may be a ring electrode.

The invention is a catheter tip. The catheter tip includes a plurality of coaxtrodes. The coaxtrodes are arranged in a pattern that is constant regardless of rotational orientation. In some embodiments, the tip includes conduction paths printed within the tip. The invention is limited by the scope of the appended independent claim 1. Preferred embodiments are disclosed in the dependent claims.

### BRIEF DESCRIPTION OF THE DRAWINGS

Figure 1 is a schematic diagram of an electrophysiology system, such as may be used in an electrophysiology study.
Figure 2 depicts an exemplary distal portion of a catheter used in an electrophysiology study.
Figure 3 is a close-up view of the distal tip of the catheter depicted in Figure 2.
Figure 4 is an enlarged view of a grid-shaped electrode assembly.
Figure 5 is an enlarged view of the distal tip of a macro scale mapping device.

### DETAILED DESCRIPTION

The present disclosure provides exemplary methods, apparatuses, and systems for the creation of electrophysiology maps (*e*.*g*., electrocardiographic maps). For purposes of illustration, several exemplary embodiments will be described in detail herein in the context of a cardiac electrophysiology procedure. It is contemplated, however, that the methods, apparatuses, and systems described herein can be utilized in other contexts.

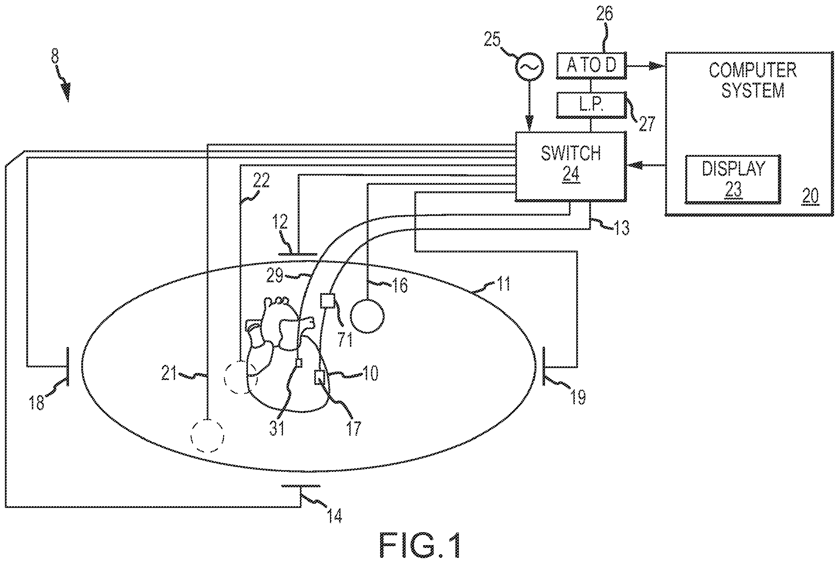

Figure 1 shows a schematic diagram of an electrophysiology system 8 for conducting cardiac electrophysiology studies by navigating a cardiac catheter and measuring electrical activity occurring in a heart 10 of a patient 11 and three-dimensionally mapping the electrical activity and/or information related to or representative of the electrical activity so measured. System 8 can be used, for example, to create an anatomical model of the patient's heart 10 using one or more electrodes. System 8 can also be used to measure electrophysiology data at a plurality of points along a cardiac surface and store the measured data in association with location information for each measurement point at which the electrophysiology data was measured, for example to create a diagnostic data map of the patient's heart 10.

As one of ordinary skill in the art will recognize, and as will be further described below, system 8 can determine the location, and in some aspects the orientation, of objects, typically within a three-dimensional space, and express those locations as position information determined relative to at least one reference.

For simplicity of illustration, the patient 11 is depicted schematically as an oval. In the embodiment shown in Figure 1, three sets of surface electrodes (e.g., patch electrodes) are shown applied to a surface of the patient 11, defining three generally orthogonal axes, referred to herein as an x-axis, a y-axis, and a z-axis. In other embodiments the electrodes could be positioned in other arrangements, for example multiple electrodes on a particular body surface. As a further alternative, the electrodes do not need to be on the body surface, but could be positioned internally to the body or on an external frame.

In Figure 1, the x-axis surface electrodes 12, 14 are applied to the patient along a first axis, such as on the lateral sides of the thorax region of the patient (e.g., applied to the patient's skin underneath each arm) and may be referred to as the Left and Right electrodes. The y-axis electrodes 18, 19 are applied to the patient along a second axis generally orthogonal to the x-axis, such as along the inner thigh and neck regions of the patient, and may be referred to as the Left Leg and Neck electrodes. The z-axis electrodes 16, 22 are applied along a third axis generally orthogonal to both the x-axis and the y-axis, such as along the sternum and spine of the patient in the thorax region, and may be referred to as the Chest and Back electrodes. The heart 10 lies between these pairs of surface electrodes 12/14, 18/19, and 16/22.

An additional surface reference electrode (*e*.*g*., a "belly patch") 21 provides a reference and/or ground electrode for the system 8. The belly patch electrode 21 may be an alternative to a fixed intra-cardiac electrode 31, described in further detail below. It should also be appreciated that, in addition, the patient 11 may have most or all of the conventional electrocardiogram ("ECG" or "EKG") system leads in place. In certain embodiments, for example, a standard set of 12 ECG leads may be utilized for sensing electrocardiograms on the patient's heart 10. This ECG information is available to the system 8 (*e*.*g*., it can be provided as input to computer system 20). Insofar as ECG leads are well understood, and for the sake of clarity in the figures, only one lead 6 and its connection to computer system 20 is illustrated in Figure 1.

A representative catheter 13 having at least one electrode 17 (*e*.*g*., a distal electrode) is also depicted in schematic fashion. This representative catheter electrode 17 can be referred to as a "measurement electrode." As described in greater detail below, multiple electrodes on catheter 13, or on multiple such catheters, will typically be used. In one embodiment, for example, system 8 may utilize sixty-four electrodes on twelve catheters disposed within the heart and/or vasculature of the patient. Of course, this embodiment is merely exemplary, and any number of electrodes and catheters may be used. Indeed, in some embodiments, a high density mapping catheter, such as the EnSite™ Array™ non-contact mapping catheter of St. Jude Medical, Inc., can be utilized.

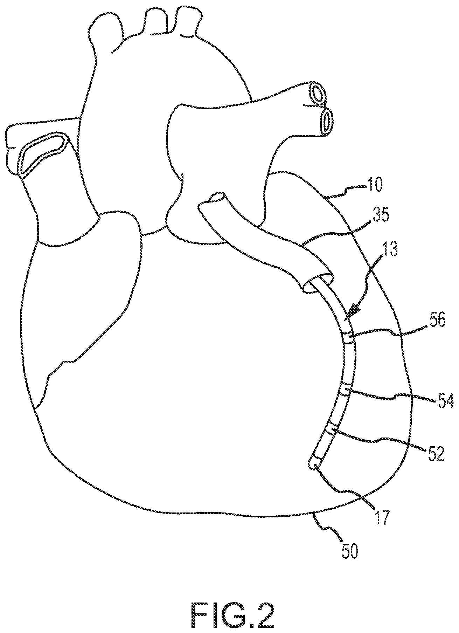

Likewise, it should be understood that catheter 13 (or multiple such catheters) are typically introduced into the heart and/or vasculature of the patient via one or more introducers and using familiar procedures. For purposes of this disclosure, a segment of an exemplary catheter 13 is shown in Figure 2. In Figure 2, catheter 13 extends into the left ventricle 50 of the patient's heart 10 through a transseptal sheath 35. The use of a transseptal approach to the left ventricle is well known and will be familiar to those of ordinary skill in the art, and need not be further described herein. Of course, catheter 13 can also be introduced into the heart 10 in any other suitable manner.

Catheter 13 includes a distal tip 17, which is described in further detail below, as well as a plurality of electrodes 52, 54, 56 spaced along its length in the illustrated embodiment. Typically, the spacing between adjacent electrodes will be known, though it should be understood that the electrodes may not be evenly spaced along catheter 13 or of equal size to each other. Since each of these electrodes 52, 54, 56 lies within the patient, location data may be collected simultaneously for each of the electrodes by system 8.

Returning now to Figure 1, in some embodiments, a fixed reference electrode 31 (e.g., attached to a wall of the heart 10) is shown on a second catheter 29. For calibration purposes, this electrode 31 may be stationary (e.g., attached to or near the wall of the heart) or disposed in a fixed spatial relationship with the measurement electrodes (e.g., electrodes 52, 54, 56, as well as electrodes on tip 17), and thus may be referred to as a "navigational reference" or "local reference." The fixed reference electrode 31 may be used in addition or alternatively to the surface reference electrode 21 described above. In many instances, a coronary sinus electrode or other fixed electrode in the heart 10 can be used as a reference for measuring voltages and displacements; that is, as described below, fixed reference electrode 31 may define the origin of a coordinate system.

Each surface electrode is coupled to a multiplex switch 24, and the pairs of surface electrodes are selected by software running on a computer 20, which couples the surface electrodes to a signal generator 25. Alternately, switch 24 may be eliminated and multiple (e.g., three) instances of signal generator 25 may be provided, one for each measurement axis (that is, each surface electrode pairing).

The computer 20, for example, may comprise a conventional general-purpose computer, a special-purpose computer, a distributed computer, or any other type of computer. The computer 20 may comprise one or more processors 28, such as a single central processing unit (CPU), or a plurality of processing units, commonly referred to as a parallel processing environment, which may execute instructions to practice the various aspects disclosed herein.

Generally, three nominally orthogonal electric fields are generated by a series of driven and sensed electric dipoles (*e*.*g*., surface electrode pairs 12/14, 18/19, and 16/22) in order to realize catheter navigation in a biological conductor. Alternatively, these orthogonal fields can be decomposed and any pairs of surface electrodes can be driven as dipoles to provide effective electrode triangulation. Likewise, the electrodes 12, 14, 18, 19, 16, and 22 (or any other number of electrodes) could be positioned in any other effective arrangement for driving a current to or sensing a current from an electrode in the heart. For example, multiple electrodes could be placed on the back, sides, and/or belly of patient 11. For any desired axis, the potentials measured across the roving electrodes resulting from a predetermined set of drive (source-sink) configurations may be combined algebraically to yield the same effective potential as would be obtained by simply driving a uniform current along the orthogonal axes.

Thus, any two of the surface electrodes 12, 14, 16, 18, 19, 22 may be selected as a dipole source and drain with respect to a ground reference, such as belly patch 21, while the unexcited electrodes measure voltage with respect to the ground reference. The electrodes 52, 54, 56 placed in the heart 10 are exposed to the field from a current pulse and are measured with respect to ground, such as belly patch 21. In practice the catheters within the heart 10 may contain more or fewer electrodes than the four shown, and each electrode potential may be measured. As previously noted, at least one electrode may be fixed to the interior surface of the heart to form a fixed reference electrode 31, which is also measured with respect to ground, such as belly patch 21, and which may be defined as the origin of the coordinate system relative to which localization system 8 measures positions. Data sets from each of the surface electrodes, the internal electrodes, and the virtual electrodes may all be used to determine the location of the electrodes 52, 54, 56 within heart 10.

The measured voltages may be used by system 8 to determine the location in three-dimensional space of the electrodes inside the heart, such as electrodes 52, 54, 56, relative to a reference location, such as reference electrode 31. That is, the voltages measured at reference electrode 31 may be used to define the origin of a coordinate system, while the voltages measured at electrodes 52, 54, 56 may be used to express the location of electrodes 52, 54, 56 relative to the origin. In some embodiments, the coordinate system is a three-dimensional (x, y, z) Cartesian coordinate system, although other coordinate systems, such as polar, spherical, and cylindrical coordinate systems, are contemplated.

As should be clear from the foregoing discussion, the data used to determine the location of the electrode(s) within the heart is measured while the surface electrode pairs impress an electric field on the heart. The electrode data may also be used to create a respiration compensation value used to improve the raw location data for the electrode locations as described in United States patent no. 7,263,397. The electrode data may also be used to compensate for changes in the impedance of the body of the patient as described, for example, in United States patent no. 7,885,707.

In one representative embodiment, the system 8 first selects a set of surface electrodes and then drives them with current pulses. While the current pulses are being delivered, electrical activity, such as the voltages measured with at least one of the remaining surface electrodes and *in vivo* electrodes, is measured and stored. Compensation for artifacts, such as respiration and/or impedance shifting, may be performed as indicated above.

In some embodiments, system 8 is the EnSite™ Velocity™ cardiac mapping and visualization system of St. Jude Medical, Inc., which generates electrical fields as described above, or another such system that relies upon electrical fields. Other systems, however, may be used in connection with the present teachings, including for example, the CARTO navigation and location system of Biosense Webster, Inc., the AURORA® system of Northern Digital Inc., or Sterotaxis' NIOBE® Magnetic Navigation System, all of which utilize magnetic fields rather than electrical fields. The localization and mapping systems described in the following patents can also be used with the present invention: United States Patent Nos. 6,990,370; 6,978,168; 6,947,785; 6,939,309; 6,728,562; 6,640,119; 5,983,126; and 5,697,377.

As described above, catheter 13, and, in particular, electrodes carried thereon (*e*.*g*., electrodes 52, 54, and 56, as well as any electrodes within distal tip 17), can be used not only to measure the location of catheter 13 within the patient's heart 10, but also to measure electrograms or tissue impedance from which various electrophysiological parameters can be extracted, including, without limitation, conduction velocity, transmembrane currents, and the like.

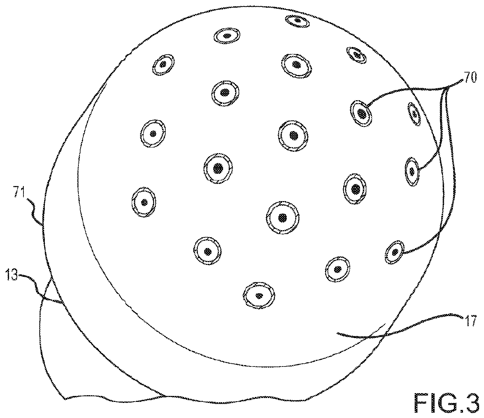

As shown in Figure 3, the distal tip 17 of catheter 13 includes a plurality of electrodes 70. Electrodes 70 may be ring electrodes and/or dot or circle electrodes (referred to herein as "spot electrodes"). Electrodes 70 are spaced about 1mm - 5mm apart, center to center.

In some embodiments, the electrodes 70 may be coaxtrodes. As used herein, the term "coaxtrode" refers to a spot electrode surrounded by a ring electrode. Advantageously, coaxtrodes are capable of providing a direction independent measure of activation time. In other words, by using coaxial electrodes, signal analysis is independent of the direction of signal propagation or tip orientation. For example, the coaxtrodes can enable determination of surface potentials within a small area following larger-scale mapping and/or signal propagation direction independent of device orientation. The coaxtrode configuration is able to provide an orientation-independent bipolar electrogram. This is in contrast to conventional ring electrode pairs that produce bipolar electrograms that are dependent on the orientation relative to the depolarization wavefront. The closely spaced electrodes of small surface area permit local tissue impedance measurements via the four electrode method.

The coaxtrodes 70 can be arranged on distal tip 17 in a hexagonal pattern. An additional coaxtrode 70 can be centrally located within the hexagon. In certain embodiments, the coaxtrodes 70 are within about 1-2 mm of each other and are in a pattern that is constant regardless of orientation. Such an arrangement of coaxtrodes allows for a large number of directly measured surface potentials within a small area.

The coaxtrodes 70 could also be configured to extract propagation direction by vector loop mapping. Epicardial potential differences are often described as a vector representation. Depending on the amount of divergence among vector angles near the maximum amplitude, loops have been classified as narrow, open or hooked. In the normal myocardium, open loops are thought to be caused by a change of direction of propagation, and hooked loops by discontinuous conduction. The distal tip as shown in Fig. 3 can be used to analyze superficial extracellular potentials during depolarization. A benefit of using coaxtrodes 70 as opposed to a high-density of ring or dot electrodes is ease of software processing with a bipole and improved information due to smaller lead field.

An amplifier 71 is operably coupled to coaxtrodes 70 in order to amplify the signals received by coaxtrodes 70. In some embodiments, amplifier 71 is located within distal tip 17, for example in a position proximal to the coaxtrodes. Alternatively, amplifier 71 can be located within a handle at the proximal end of catheter 13, or even external to catheter 13.

Amplifier 71 can be multiplexed to all of the coaxtrodes. In other aspects, a unique amplifier is operably coupled to each coaxtrode. Indeed, both one-to-one and one-to-many correspondence between amplifiers 71 and coaxtrodes 70 are contemplated.

Conductors from the coaxtrodes 70 to amplifier(s) 71 may be configured as a twisted pair, coaxial, triaxial or shielded twisted pair in order to mitigate noise. In the case of triaxial or shielded twisted pair, the outermost shield could be tied to a ring conductor or other large surface area electrode submerged in a blood pool.

Distal tip 17, including the electrodes 70 (*e*.*g*., coaxtrodes), can be made from a flexible circuit.

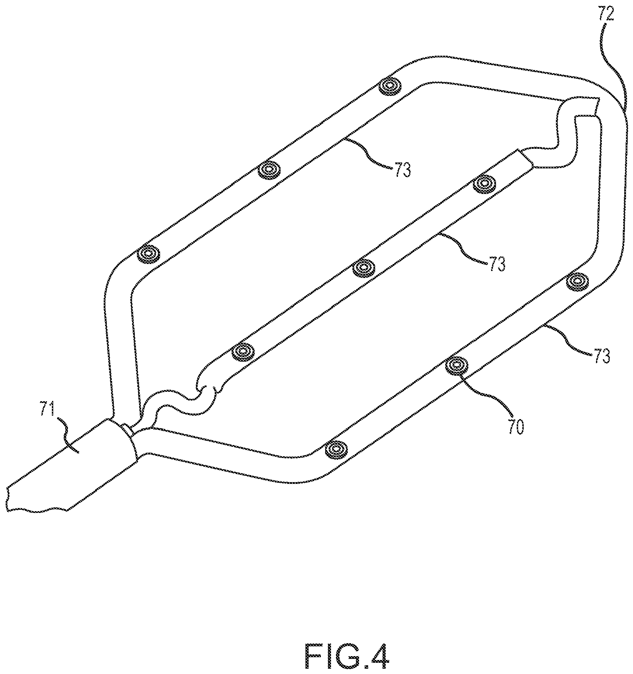

Figure 4 illustrates an example, not part of the invention, wherein a grid-shaped electrode assembly 72 is utilized in place of distal tip 17. The assembly 72 is shown having three splines 73, however the assembly 72 may have any number of splines 73 without departing from the present teachings. Likewise, the embodiment shown in Figure 4 has nine coaxtrodes 70, but can contain more or fewer than nine coaxtrodes 70.

In an example, not part of the invention, a segura basket or a D-shaft in a spiral shape contains the coaxtrodes and is utilized instead of distal tip 17.

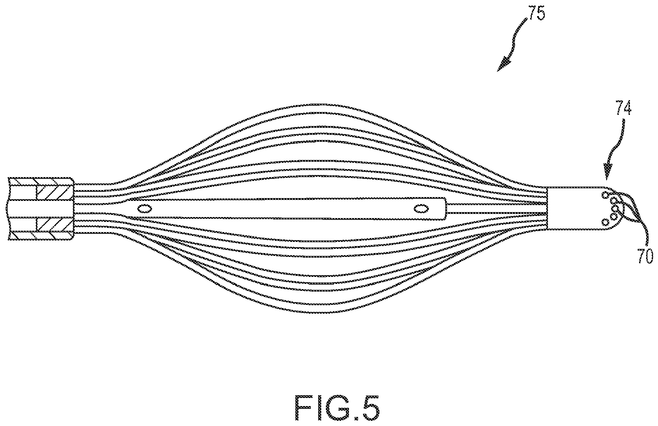

Figure 5 illustrates an embodiment wherein coaxtrodes 70 are arranged on the distal tip 74 of a macro scale mapping device 75, such as the EnSite™ Array™ non-contact mapping catheter of St. Jude Medical. The coaxtrodes 70 can be configured for contact mapping, which can be performed simultaneously with the noncontact global mapping. A benefit of such a device is the ability to achieve high resolution in a region of interest (e.g., using the coaxtrodes to gather electrophysiology data) while also being able to provide a chamber-wide image (e.g., using the non-contact aspects of the catheter). Indeed, this configuration is advantageous because mechanistic understanding of the spatiotemporal phenomena underlying cardiac arrhythmias calls for both micro- and macro scale imaging. The requirement for combined macro-micro-examination is of particular importance for phenomena occurring at fine spatial scales or in a heterogeneous setting. Examples include the activity at the core of a macroscopic spiral wave, cell-level phenomena during macroscopic wave meandering and wavebreaks in fibrillation-like conditions, and coupling and propagation between discrete structures of cell populations. In other embodiments, the contact and noncontact mapping can be performed sequentially.

All directional references (e.g., upper, lower, upward, downward, left, right, leftward, rightward, top, bottom, above, below, vertical, horizontal, clockwise, and counterclockwise) are only used for identification purposes to aid the reader's understanding of the present invention. Joinder references (e.g., attached, coupled, connected, and the like) are to be construed broadly and may include intermediate members between a connection of elements and relative movement between elements. As such, joinder references do not necessarily infer that two elements are directly connected and in fixed relation to each other.
It is intended that all matter contained in the above description or shown in the accompanying drawings shall be interpreted as illustrative only and not limiting. Changes in detail or structure may be made without departing from the scope of the invention as defined in the appended claims.

## Claims

1. A catheter tip, the tip comprising a proximal end and a distal end wherein the distal end comprises an exposed continuous end surface, and further wherein the exposed continuous end surface comprises a plurality of coaxtrodes (70), wherein the plurality of coaxtrodes (7) are arranged on the distal end in a hexagonal pattern on the exposed continuous end surface, wherein a coaxtrode (70) is a spot electrode surrounded by a ring electrode.

2. The catheter tip of claim 1, wherein the tip further comprises conduction paths printed within the tip.

3. An electrophysiology apparatus, the apparatus comprising:
a catheter (13) comprising a body having a proximal end and a distal end, the distal end having a catheter tip (17) according to claims 1 or 2; and
a signal processor operably connected to the plurality of coaxtrodes (70), wherein the signal processor measures at least one electrophysiological parameter.

4. The electrophysiology apparatus of claim 3, wherein the at least one electrophysiological parameter is selected from the group consisting of, transmembrane current, tissue impedance, and local conduction velocity.

5. The apparatus of claim 3, wherein the distal tip has a proximal end and a distal end, and wherein at least one amplifier is located at the proximal end of the tip, and wherein the at least one amplifier is operably connected to the plurality of coaxtrodes (70).

6. The apparatus of claim 3, wherein the catheter is operably attached to a handle, and wherein at least one amplifier is located in the catheter handle, and wherein the at least one amplifier is operably connected to the plurality of coaxtrodes (70).

7. The apparatus of any one of claims 3 to 6, wherein the plurality of coaxtrodes (70) comprises seven coaxtrodes (70), wherein six of the coaxtrodes (70) define a hexagon and the seventh coaxtrode is positioned internal to the hexagon.

8. The apparatus of claim 7, wherein the seventh coaxtrode is located centrally in the hexagon.

9. The apparatus of any one of claims 3 to 8, wherein the catheter (13) comprises a localization element located proximal to the distal tip (17).

10. The apparatus of claim 9, wherein the localization element comprises a ring electrode.

11. The apparatus according to claim 3, further comprising a basket-shaped assembly operably connected to the distal tip region, wherein a plurality of coaxtrodes (70) are additionally located on the basket-shaped assembly.

## Patentansprüche

1. Katheterspitze, bei der die Spitze ein proximales Ende und ein distales Ende aufweist, wobei das distale Ende eine freigelegte, durchgehende Stirnfläche aufweist, und wobei ferner die freigelegte, durchgehende Stirnfläche eine Mehrzahl von Koaxtroden (70) aufweist, wobei die Mehrzahl der Koaxtroden (70) auf dem distalen Ende in einem hexagonalen Muster auf der freigelegten, durchgehenden Stirnfläche angeordnet sind, wobei eine Koaxtrode (70) eine Punktelektrode ist, die von einer Ringelektrode umgeben ist.

2. Katheterspitze nach Anspruch 1, bei der die Spitze ferner Leitungspfade aufweist, die in die Spitze gedruckt sind.

3. Elektrophysiologische Vorrichtung, wobei die Vorrichtung aufweist:
einen Katheter (13) mit einem Körper, der ein proximales Ende und ein distales Ende aufweist, wobei das distale Ende eine Katheterspitze (17) nach Anspruch 1 oder 2 aufweist; und
einen Signalprozessor, der operativ mit der Mehrzahl der Koaxtroden (70) verbunden ist, wobei der Signalprozessor mindestens einen elektrophysiologischen Parameter misst.

4. Elektrophysiologische Vorrichtung nach Anspruch 3, bei der der mindestens eine elektrophysiologische Parameter ausgewählt ist aus der Gruppe mit Transmembranstrom, Gewebeimpedanz und lokale Leitgeschwindigkeit.

5. Vorrichtung nach Anspruch 3, bei der das distale Ende ein proximales Ende und ein distales Ende aufweist, und mindestens ein Verstärker an dem proximalen Ende der Spitze vorgesehen ist und der mindestens eine Verstärker operativ mit der Mehrzahl von Koaxtroden (70) verbunden ist.

6. Vorrichtung nach Anspruch 3, bei der der Katheter operativ an einem Griff angebracht ist, und mindestens ein Verstärker in dem Kathetergriff vorgesehen ist und der mindestens eine Verstärker operativ mit der Mehrzahl von Koaxtroden (70) verbunden ist.

7. Vorrichtung nach einem der Ansprüche 3 bis 6, bei der die Mehrzahl der Koaxtroden (70) sieben Koaxtroden (70) aufweist, wobei sechs der Koaxtroden (70) ein Hexagon definieren, und die siebte Koaxtrode in dem Hexagon positioniert ist.

8. Vorrichtung nach Anspruch 7, bei der die siebente Koaxtrode sich zentral in dem Hexagon befindet.

9. Vorrichtung nach einem der Ansprüche 3 bis 8, bei der der Katheter (13) ein Lokalisierungselement aufweist, das proximal zu der distalen Spitze (17) vorgesehen ist.

10. Vorrichtung nach Anspruch 9, bei der das Lokalisierungselement eine Ringelektrode aufweist.

11. Vorrichtung nach Anspruch 3, ferner mit einem korbförmigen Aufbau, der operativ mit der distalen Spitzenregion verbunden ist, wobei eine Mehrzahl von Koaxtroden (70) sich zusätzlich an dem korbförmigen Aufbau befindet.

## Revendications

1. Pointe de cathéter, la pointe comprenant une extrémité proximale et une extrémité distale dans laquelle l'extrémité distale comprend une surface d'extrémité continue exposée, et en outre dans laquelle la surface d'extrémité continue exposée comprend une pluralité d'électrodes coaxiales (70), dans laquelle la pluralité d'électrodes coaxiales (7) est disposée sur l'extrémité distale dans un motif hexagonal sur la surface d'extrémité continue exposée, dans laquelle une électrode coaxiale (70) est une électrode ponctuelle entourée d'une électrode annulaire.

2. Pointe de cathéter de la revendication 1, dans laquelle la pointe comprend en outre des chemins de conduction imprimés dans la pointe.

3. Appareil d'électrophysiologie, l'appareil comprenant :
un cathéter (13) comprenant un corps ayant une extrémité proximale et une extrémité distale, l'extrémité distale ayant une pointe de cathéter (17) selon la revendication 1 ou 2 ; et
un processeur de signal fonctionnellement connecté à la pluralité d'électrodes coaxiales (70), dans lequel le processeur de signal mesure au moins un paramètre électrophysiologique.

4. Appareil d'électrophysiologie de la revendication 3, dans lequel ledit au moins un paramètre électrophysiologique est choisi parmi le groupe consistant en un courant transmembranaire, une impédance de tissu et une vitesse de conduction locale.

5. Appareil de la revendication 3, dans lequel la pointe distale présente une extrémité proximale et une extrémité distale, et dans lequel au moins un amplificateur est localisé à l'extrémité proximale de la pointe, et dans lequel au moins un amplificateur est fonctionnellement connecté à la pluralité d'électrodes coaxiales (70).

6. Appareil de la revendication 3, dans lequel le cathéter est fonctionnellement connecté à une poignée, et dans lequel au moins un amplificateur est localisé dans la poignée de cathéter et dans lequel ledit au moins un amplificateur est fonctionnellement connecté à la pluralité d'électrodes coaxiales (70).

7. Appareil de l'une quelconque des revendications 3 à 6, dans lequel la pluralité d'électrodes coaxiales (70) comprend sept électrodes coaxiales (70), dans lequel six des électrodes coaxiales (70) définissent un hexagone et la septième électrode coaxiale est placée à intérieur de l'hexagone.

8. Appareil de la revendication 7, dans lequel la septième électrode coaxiale est localisée centralement dans l'hexagone.

9. Appareil de l'une quelconque des revendications 3 à 8, dans lequel le cathéter (13) comprend un élément de localisation localisé proche de la pointe distale (17).

10. Appareil de la revendication 9, dans lequel l'élément de localisation comprend une électrode annulaire.

11. Appareil selon la revendication 3, comprenant en outre un assemblage en forme de panier fonctionnellement connecté à la zone de pointe distale, dans lequel une pluralité d'électrodes coaxiales (70) est de plus localisée sur l'assemblage en forme de panier.
